(19) Europäisches Patentamt
European Patent Office
Office européen des brevets

(11) Veröffentlichungsnummer: 0 126 030
A2

(12) EUROPÄISCHE PATENTANMELDUNG

(21) Anmeldenummer: 84810219.0

(22) Anmeldetag: 08.05.84

(51) Int. Cl.³: C 07 D 277/74
C 07 D 235/28

(30) Priorität: 14.05.83 GB 8313321

(43) Veröffentlichungstag der Anmeldung: 21.11.84 Patentblatt 84/47

(84) Benannte Vertragsstaaten: AT DE FR GB IT

(71) Anmelder: CIBA-GEIGY AG
Postfach
CH-4002 Basel(CH)

(72) Erfinder: Bosshard, Hans
Hohe Winde-Strasse 23
CH-4059 Basel(CH)

(72) Erfinder: Greuter, Hans, Dr.
Grendel 412
CH-5268 Eiken(CH)

# (54) Verfahren zur Herstellung von beta-(Benzthiazolylthio)- und beta-(Benzimidazolylthio)-carbonsäuren.

(57) Die Reaktion von 2-Mercapto-benzthiazol oder -benzimidazol mit α,ß-ungesättigten Carbonsäuren in stark saurem Reaktionsmedium liefert Verbindungen der Formel I

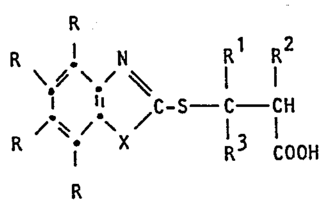

I,

worin X Schwefel oder NH ist, jedes R unabhängig voneinander H, Alkyl, Halogenalkyl, alkoxy, Alkylthio, Alkylsulfonyl, Phenyl, Alkylphenyl, Phenylalkyl, Cycloalkyl, Halogen, $NO_2$, CN, COOH, COOAlkyl, OH oder eine Amino- oder Carbamoylgruppe bedeutet und $R^1$, $R^2$ und $R^3$ unabhängig voneinander H, Alkyl, Halogenalkyl, Hydroxyalkyl, Alkoxyalkyl, Carboxyalkyl, Carboxyl, unsubstituiertes oder substituiertes Aryl oder Aralkyl bedeuten oder $R^1$ und $R^2$ zusammen unverzweigtes oder verzweigtes Alkylen bedeuten, das durch 1 oder 2 Carboxylgruppen substituiert sein kann.

EP 0 126 030 A2

CIBA-GEIGY AG
Basel (Schweiz) 3-14427/+

Verfahren zur Herstellung von β-(Benzthiazolylthio)- und β-(Benzimidazolylthio)-carbonsäuren

Die vorliegende Erfindung betrifft ein Verfahren zur Herstellung von aliphatischen oder cycloaliphatischen Carbonsäuren welche in β-Stellung durch einen heterocyclischen Mercaptorest substituiert sind und das dadurch gekennzeichnet ist, dass man eine α,β-ungesättigte Carbonsäure in stark saurem Medium mit einem heterocyclischen Mercaptan reagieren lässt.

Die Addition von Mercaptanen an α,β- ungesättigte Säuren ist prinzipiell bekannt. Sie wird jedoch üblicherweise in einem basischen Medium oder unter Verwendung basischer Katalysatoren ausgeführt. Man nimmt an, dass dabei der erste Schritt in einer Anlagerung des Mercaptid-Anions an das β-C-Atom der Carbonsäure besteht. F.B. Ziently et al. (J. Org. Chem. 27 (1962), 3140) beschrieben die Addition von verschiedenen Thiolen an Maleinsäureanhydrid unter basischer Katalyse. Diese Autoren bemerken, dass die Addition bei radikalischer Katalyse nur mässige Ausbeuten ergibt und dass Lewis-Saäuren keine katalysierende Wirkung haben.

Im US-Patent 2,725,364 wird erwähnt, dass sich Malein- oder Fumarsäure in wässrig-alkalischer Lösung an 2-Mercaptobenzthiazol bei 30 - 60°C addieren lässt, wozu dort aber keine experimentellen Details beschrieben werden.

Eigene Versuche zur Addition α,β-ungesättigter Carbonsäuren und insbesondere von Malein- und Fumarsäure an 2-Mercaptobenzthiazol in alkalisch-wässrigem Medium bei 45 - 50°C zeigten jedoch, dass

innerhalb 100 Stunden keine Addition eintritt. Ueberraschenderweise wurde jedoch gefunden, dass in stark saurem Medium die Addition glatt verläuft und die entsprechenden β-Benzthiazolyl-2-mercaptocarbonsäuren in hoher Ausbeute und Reinheit entstehen. Dasselbe gilt für die Addition an 2-Mercaptobenzimidazol.

Gegenstand der Erfindung ist daher ein Verfahren zur Herstellung von Verbindungen der Formel I,

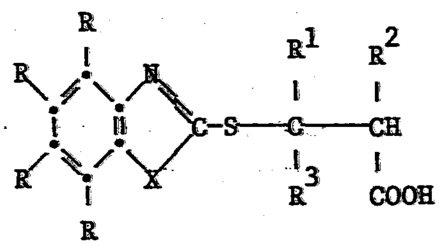

I ,

worin X Schwefel oder NH bedeutet, jedes R unabhängig von den anderen Wasserstoff, Alkyl, Halogenalkyl, Alkoxy, Alkylthio, Alkylsulfonyl, Phenyl, Alkylphenyl, Phenylalkyl, Cycloalkyl, Halogen, $-NO_2$, -CN, -COOH, -COOAlkyl, -OH oder eine primäre, sekundäre oder tertiäre Amino- oder Carbamoylgruppe bedeutet, und $R^1$, $R^2$, $R^3$ und $R^4$ unabhängig voneinander Wasserstoff, Alkyl, Halogenalkyl, Hydroxyalkyl, Alkoxyalkyl, Carboxyalkyl, Carboxyl, unsubstituiertes oder substituiertes Aryl oder Aralkyl bedeuten oder $R^1$ und $R^2$ zusammen eine direkte Bindung oder unverzweigtes oder verzweigtes Alkylen bedeuten, das durch 1 oder 2 Carboxylgruppen substituiert sein kann, durch Reaktion eines Mercaptans der Formel II

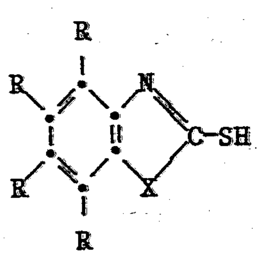

II

mit einer ungesättigten Carbonsäure der Formel III

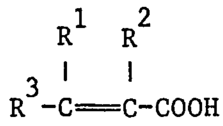 III

oder deren Anhydrid in stark saurem Medium.

Wenn in Formel I und II R Alkyl ist, so kann dies unverzweigtes oder verzweigtes Alkyl sein und ist vorzugsweise $C_1$-$C_{12}$-Alkyl wie z.B. Methyl, Ethyl, Propyl, Isopropyl, n-Butyl, Isobutyl, tert.-Butyl, Pentyl, Hexyl, Octyl, Nonyl, Decyl oder Dodecyl. R als Halogenalkyl ist vorzugsweise $C_1$-$C_4$-Halogenalkyl, wie z.B. Chlormethyl, Mono-, Di-, Trifluormethyl, Trichlormethyl oder 2-Chlorethyl. R als Alkoxy oder Alkylthio hat vorzugsweise 1-4 C-Atome und kann z.B. Methoxy, Ethoxy, Isopropoxy, Methylthio, Propylthio oder tert.-Butylthio sein. R als Alkylsulfonyl ist vorzugsweise $C_1$-$C_{12}$-Alkylsulfonyl und kann z.B. Methylsulfonyl, tert.-Butylsulfonyl, n-Octylsulfonyl oder n-Dodecylsulfonyl sein.

R als Cycloalkyl enthält vorzugsweise 5-8 C-Atome. Beispiele hierfür sind Cyclopentyl, Cyclohexyl oder Cyclooctyl. R als Alkylphenyl oder Phenylalkyl hat vorzugsweise 7 - 12 C-Atome und kann z.B. Tolyl, Xylyl, Ethylphenyl, tert.-Butylphenyl, Benzyl, 1- oder 2-Phenylethyl oder $\alpha,\alpha$-Dimethylbenzyl sein. R als -COOAlkyl ist vorzugsweise -COO($C_1$-$C_4$-Alkyl) wie z.B. Methoxycarbonyl, Ethoxycarbonyl oder Butoxycarbonyl. R als primäre, sekundäre oder tertiäre Aminogruppe oder Carbamoylgruppe ist vorzugsweise eine solche mit bis zu 20 C-Atomen wie z.B. $-NH_2$, $-NHCH_3$, $-NH-C_4H_9$, -NH-Phenyl, -NH-Cyclohexyl, $-N(CH_3)_2$, $-N(C_2H_5)_2$, $-N(i-C_3H_7)_2$, $-N(CH_2CH_2OH)_2$, $-N(C_4H_9)_2$, $-N(C_8H_{17})_2$, $-N(CH)_3$-Phenyl, $-N(CH_3)$-Benzyl, Piperidino, Morpholino, $-CONH_2$, $-CONHCH_3$, -CONHPhenyl, $-CON(CH_3)_2$, $-CON(C_6H_{13})_2$, Morpholinocarbonyl oder Piperidinocarbonyl.

Vorzugsweise verwendet man eine Verbindung der Formel II, worin mindestens zwei der Substituenten R Wasserstoff sind, insbesondere Verbindungen der Formel II, worin ein R Wasserstoff, $C_1$-$C_4$-Alkyl, $C_1$-$C_4$-Alkoxy, Halogen oder -COOH ist und die anderen drei R Wasserstoff sind.

Bevorzugt verwendet man Verbindungen der Formel II, worin X Schwefel ist und erhält dabei die entsprechenden β-(Benzthiazol-2-ylthio)carbonsäuren.

Wenn in Formel I und III die Substituenten $R^1$, $R^2$ oder $R^3$ Alkyl bedeuten, so können sie unverzweigtes oder verzweigtes Alkyl bedeuten, insbesondere solches mit 1 - 12 C-Atomen. Beispiele hierfür sind Methyl, Ethyl, n-Propyl, Isopropyl, n-Butyl, sec.-Butyl, Isobutyl, n-Pentyl, Isopentyl, Hexyl, Heptyl, Octyl, Nonyl, Decyl oder Dodecyl. Sind $R^1$, $R^2$ oder $R^3$ Halogenalkyl oder Hydroxyalkyl, so haben sie vorzugsweise 1-4 C-Atome. Beispiele hierfür sind Hydroxymethyl, 1- oder 2-Hydroxyethyl, 1-, 2- oder 3-Hydroxypropyl, 3-Hydroxybutyl, Chlormethyl, Mono-, Di- oder Trifluormethyl, Brommethyl, 2-Chlorethyl, 3-Chlorpropyl oder 2-Chlorbutyl.

$R^1$, $R^2$ und $R^3$ als Alkoxyalkyl können insbesondere $C_2$-$C_{10}$-Alkoxyalkyl sein, wie z.B. Methoxymethyl, 1- oder 2-Methoxyethyl, Ethoxymethyl, 2-Butoxyethyl oder Octyloxymethyl. $R^1$, $R^2$ und $R^3$ als Carboxyalkyl können insbesondere $C_2$-$C_{12}$-Carboxyalkyl sein, wie z.B. Carboxymethyl, 1- oder 2-Carboxyethyl, 2- oder 3-Carboxypropyl, 1- oder 4-Carboxybutyl oder 6-Carboxyhexyl. $R^1$, $R^2$ und $R^3$ als substituiertes Aryl oder Aralkyl können insbesondere durch Halogen, Nitro, Alkyl, Alkyl, Hydroxy, Alkoxy oder Carboxy substituiertes Phenyl oder Benzyl sein, wie z.B. 4-Chlorphenyl, 3-Nitrophenyl, Tolyl, Xylyl, 4-tert.-Butylphenyl, 4-Hydroxyphenyl, 3-Methoxyphenyl, 3- oder 4-Carboxyphenyl, 4-Fluorbenzyl oder 4-Methylbenzyl.

Wenn $R^1$ und $R^2$ zusammen unverzweigtes oder verzweigtes Alkylen bedeuten, so bilden sie zusammen mit den C-Atomen, an die sie gebunden sind, einen Cycloalkanring, vorzugsweise einen Cyclopentan- oder Cyclohexanring, der durch Alkylgruppen, vorzugsweise $C_1$-$C_4$-Alkylgruppen oder durch ein oder zwei Carboxylgruppen substituiert sein kann.

Bevorzugt sind $R^1$, $R^2$ und $R^3$ unabhängig voneinander Wasserstoff $C_1$-$C_8$-Alkyl, $C_2$-$C_8$-Carboxyalkyl, Carboxy oder Phenyl oder $R^1$ und $R^2$ zusammen Tri- oder Tetramethylen. Besonders bevorzugt sind mindestens zwei der Substituenten $R^1$, $R^2$ und $R^3$ Wasserstoff.

Die Mercaptane der Formel II sind bekannte Verbindungen oder können in Analogie zu diesen hergestellt werden. Beispiele für erfindungsgemäss verwendbare Verbindungen der Formel II sind:
2-Mercaptobenzthiazol, 5-Methyl-2-mercaptobenzthiazol, 4-Isopropyl-2-mercaptobenzthiazol, 7-t-Butyl-2-mercapto-benzthiazol, 6-Cyclohexyl-2-mercapto-benzthiazol, 7-Benzyl-2-mercapto-benzthiazol, 5-Trifluormethyl-2-mercapto-benzthiazol, 6-Methoxy-2-mercapto-benzthiazol, 7-Ethoxy-2-mercaptobenzthiazol, 4-Methylthio-2-mercapto-benzthiazol, 6-Methylsulfonyl-2-mercaptobenzthiazol, 4-Fluor-2-mercapto-benzthiazol, 5-Chlor-2-mercapto-benzthiazol, 7-Brom-2-mercapto-benzthiazol, 6-Chlor-2-mercapto-benzthiazol, 4-Phenyl-2-mercapto-benzthiazol, 6-Nitro-2-mercapto-benzthiazol, 5-Cyano-2-mercapto-benzthiazol, 5-Carboxy-2-mercapto-benzthiazol, 5-Methoxycarbonyl-2-mercapto-benzthiazol, 7-Hydroxy-2-mercapto-benzthiazol, 6-Amino-2-mercapto-benzthiazol, 5-Dimethylamino-2-mercapto-benzthiazol, 5-Morpholino-2-mercapto-benzthiazol, 5-Carbamoyl-2-mercapto-benzthiazol, 5-Phenylcarbamoyl-2-mercapto-benzthiazol, 5-Chlor-6-n-butyl-2-mercapto-benzthiazol, 5-Nitro-6-n-propyl-2-mercapto-benzthiazol, 5-Brom-6-n-propoxy-2-mercapto-benzthiazol, 4,5,6-Triethyl-2-mercapto-benzthiazol, 4,5,6,7-Tetramethyl-2-mercapto-benzthiazol, 4-Methoxy-6-hydroxy-2-mercapto-benzthiazol, 4,5-Dimethyl-7-propoxy-2-mercapto-benzthiazol, 2-Mercaptobenzimidazol, 6-Methyl-2-mercapto-benzimidazol, 4-Isopropyl-2-mercapto-benzimidazol, 5-n-Hexyl-2-mercapto-benzimidazol, 6-(1,1,3,3-Tetramethylbutyl)-2-mercapto-benzimidazol, 7-Benzyl-2-mercapto-benzimidazol, 6-Ethoxy-2-mercapto-benzimidazol, 6-Isopropoxy-2-mercapto-benzimidazol, 4-Fluor-2-mercapto-benzimidazol, 5-Chlor-2-mercapto-benzimidazol, 5-Chlor-2-mercapto-benzimidazol, 5-Cyano-2-mercapto-benzimidazol, 4-Phenyl-2-mercapto-benzimidazol, 6-Nitro-2-mercapto-benzimidazol,

5-Carboxy-2-mercapto-benzimidazol, 5-Butoxycarbonyl-2-mercapto-benzimidazol, 7-Hydroxy-2-mercapto-benzimidazol, 6-Amino-, 5-Dimethylamino-, 4-Piperidino-, 5-Methylcarbamoyl- oder 5-Diethylcarbamoyl-2-mercapto-benzimidazol, 4-Brom-5-n-hexyl-2-mercapto-benzimidazol, 5-Nitro-6-n-propyl-2-mercapto-benzimidazol, 4,5,6-Triethyl-2-mercapto-benzimidazol oder 4,5-Dimethyl-7-propoxy-2-mercapto-benzimidazol.

Beispiele von ungesättigten Carbonsäuren der Formel III sind: Acrylsäure, Methacrylsäure, Crotonsäure, 2,3- oder 3,3-Dimethylacrylsäure, Propiolsäure, Phenylpropiolsäure, Maleinsäure, Fumarsäure, Acetylendicarbonsäure, Itaconsäure, Cyclohexen-1,2-dicarbonsäure, 3-Methylcyclohexen-1,2-dicarbonsäure, Ethylentetracarbonsäure, Mesaconsäure, Glutaconsäure, Aconitsäure, Citraconsäure, α-Methylenglutarsäure, α-Methylendipinsäure, α-Ethyliden-adipinsäure, Propylen-1,3-dicarbonsäure, 1-Buten-1,4-dicarbonsäure, 1-Buten-2,3,4-tricarbonsäure, 2-Pentensäure, 2-Hexensäure, 2-Octensäure, 2-Decensäure, 2-Undecensäure, 2-Dodecensäure, 2-Octadecensäure, Zimtsäure, α-Phenylacrylsäure, α-Phenylcrotonsäure, β-Benzylacrylsäure, Benzylidenmalonsäure, α-Methylzimtsäure, 4-Chlorzimtsäure oder 3-Nitrozimtsäure.

Anstelle der Carbonsäure können auch deren Anhydride verwendet werden. Bei Ausführung der Reaktion in wässrigem Medium erhält man als Produkt die Carbonsäure der Formel I. Bei Ausführung in wasserfreiem Medium erhält man zunächst das entsprechende Anhydrid, das anschliessend leicht zur Carbonsäure hydrolysiert werden kann. Eine solche Hydrolyse tritt meist bereits beim Verdünnen mit Wasser ein. Die als Reaktionskomponente verwendeten Anhydride können solche einer Monocarbonsäure sein, wie z.B. Acryl- oder Methacrylsäureanhydrid. Von besonderem Interesse ist die Verwendung der Anhydride im Falle von 1,2-Dicarbonsäuren. Beispiele für solche Anhydride sind Malein-, Itacon- und Citraconsäureanhydrid, sowie Cyclohexen-1,2-dicarbonsäure-anhydrid.

Bevorzugt verwendet man als Carbonsäure eine Di- oder Polycarbonsäure der Formel III, worin $R^3$ Carboxyl ist oder $R^2$ Carboxymethyl ist, oder das cyclische Anhydrid einer solchen Verbindung. Besonders bevorzugt verwendet man Maleinsäure oder Maleinsäureanhydrid.

Die Reaktion von II mit III wird in stark saurem Medium ausgeführt. Das Reaktionsmedium kann z.B. eine wässrige Lösung einer Mineralsäure sein wie z.B. von $H_2SO_4$, $H_3PO_4$, HCl, HBr, $HBF_4$, $HClO_4$, $H_2S_2O_7$ oder Polyphosphorsäure. Organische Säuren wie z.B. Ameisensäure, Trifluoressigsäure oder p-Toluolsulfonsäuren können in wässriger Lösung oder in organischer Lösung eingesetzt werden. Bestimmte Säuren können auch in unverdünnter Form als Reaktionsmedium dienen, z.B. Trifluoressigsäure, Ameisensäure oder Phosphorsäure.

Als Säuren können auch Lewis-Säuren verwendet werden, wie z.B. $AlCl_3$, $AlBr_3$, $BF_3$, $SbF_5$, $SbCl_5$ oder $SnCl_4$. In diesem Fall führt man die Reaktion in einem inerten Lösungsmittel aus, in dem die Lewis-Säure löslich ist, beispielsweise in Diethylether oder in halogenierten Kohlenwasserstoffen.

Wenn die Ausgangsmaterialien in der verwendeten wässrigen Säure nicht löslich sind, so kann man auch ein mit Wasser mischbares organisches Lösungsmittel zusetzen, beispielsweise Methanol, Ethanol, Ethylenglykolmonomethylether, Essigsäure, Propionsäure, Tetramethylensulfon (Sulfolan), Tetrahydrofuran, Dioxan, Aceton oder Dimethylsulfoxid. In diesem Fall arbeitet man also in einem sauren wässrigorganischen Medium.

Bevorzugt führt man die Reaktion in einer wässrigen oder wässrigorganischen Lösung einer starken Protonsäure aus, insbesondere in 60-90 %iger Schwefelsäure oder in 25-38 %iger Salzsäure.

Die Reaktionstemperatur kann im Bereich von -30°C bis zum Siedepunkt des Reaktionsmediums liegen, vorzugsweise arbeitet man bei 0° bis 100°C. Unter bestimmten Bedingungen kann es vorteilhaft sein, die

Reaktion unter Ueberdruck auszuführen, jedoch ist dies nicht notwendig.

Die Reaktionskomponenten werden im annähernden Molverhältnis 1:1 eingesetzt, wobei man die Carbonsäure III in geringem Ueberschuss einsetzt, bis zu etwa 10 Mol-%. Man kann zuerst die eine Komponente im sauren Reaktionsmedium lösen oder dispergieren und dann die zweite Komponente zugeben. Oder man mischt zuerst die beiden Komponenten und trägt dieses Gemisch langsam in das saure Reaktionsmedium ein.

Die Isolierung der Produkte kann nach üblichen Methoden erfolgen. Beim Arbeiten in einer konzentrierten Mineralsäure ist es zweckmässig, nach Beendigung der Reaktion das Reaktionsgemisch mit Wasser zu verdünnen und einen Teil der Mineralsäure durch Zugabe einer Base wie NaOH oder $NaCO_3$ zu neutralisieren, wobei das Produkt nach dem Abkühlen meist ausfällt oder durch Extraktion isoliert werden kann. Das rohe Produkt kann durch Umfällung aus einer wässrigen Base gereinigt werden. Im allgemeinen fallen gemäss dem erfindungsgemässen Verfahren die Produkte in hoher Reinheit aus, so dass oft eine weitere Reinigung unnötig ist.

Die Verbindungen der Formel I können als Korrosionsinhibitoren in wässrigen Systemen oder in Ueberzugsmitteln für Metalle verwendet werden. Die Erfindung wird durch die nachfolgenden Beispiele näher erläutert. Die Temperaturen werden darin in °C angegeben.

Beispiel 1: In 40 ml 70 %iger Schwefelsäure werden 16,8 g fein gepulvertes 2-Mercaptobenzthiazol suspendiert und 10,2 g gepulvertes Maleinsäureanhydrid innerhalb 1 Stunde unter Rühren bei 48-51° zugegeben. Nach einer weiteren Stunde bei 50° wird die Reaktionsmischung auf Raumtemperatur gekühlt und durch tropfenweise Zugabe von 250 ml Wasser bei 25-35° verdünnt. Nach 1 Stunde wird das ausgefallene Produkt abfiltriert und in verdünnter Natronlauge gelöst.

Die Lösung wird filtriert und mit Salzsäure angesäuert. Der Niederschlag wird abfiltriert und im Vakuum bei 60° getrocknet. Man erhält so 24,7 g (87 % d.Th.) Benzthiazol-2-ylthiobernsteinsäure, die bei 175-176° unter Zersetzung schmilzt.

| | | | | | |
|---|---|---|---|---|---|
| Analyse ($C_{11}H_9NO_4S_2$) | ber. | 46,64 % C | 3,18 % H | 4,94 % N | 22,61 % S |
| | gef. | 46,6 % C | 3,4 % H | 5,0 % N | 22,5 % S. |

Wird dieselbe Umsetzung mit 12,1 g Maleinsäure anstelle des Anhydrids ausgeführt, so erhält man 22, 6 g (80 % d.Th.) an Benzthiazolylthiobernsteinsäure.

Wird dieselbe Umsetzung mit 12,1 g Fumarsäure ausgeführt und lässt man die Komponenten 12 Stunden bei 40° reagieren, so erhält man 21,1 g (75 % d.Th.) an Benzthiazolylthiobernsteinsäure.

Beispiel 2: Eine fein gepulverte Mischung von 16,8 g 2-Mercaptobenzthiazol und 10,3 g Maleinsäureanhydrid werden in eine Mischung von 60 ml 36 %iger Salzsäure und 60 ml 99 %iger Essigsäure unter Rühren zugegeben. Dann erwärmt man das Reaktionsgemisch 4 Stunden auf 70 - 75° und giesst die Lösung in 500 ml Wasser. Der Niederschlag wird abfiltriert, mit kaltem Wasser gewaschen und getrocknet. Man erhält 24 g (85 % d.Th.) Benzthiazol-2-ylthiobernsteinsäure.

Beispiel 3: 3,4 g 2-Mercaptobenzthiazol und 2,0 g Maleinsäureanhydrid werden in 40 ml Trifluoressigsäure während 12 Stunden unter Rühren auf 50° erhitzt. Das Reaktionsgemisch wird mit Eiswasser verdünnt und das ausgefällte Produkt in Ethylacetat aufgenommen. Die Lösung wird dreimal mit Wasser gewaschen und zur Trockne eingedampft. Man erhält 4,5 g (79 % d.Th.) Benzthiazol-2-ylthiobernsteinsäure.

Beispiel 4: In 50 ml 70 %iger Schwefelsäure werden 16,8 gepulvertes 2-Mercaptobenzthiazol suspendiert und 13,7 g Itaconsäure werden innerhalb 30 Minuten bei 40-44° in diese Suspension eingerührt. Nach weiteren 1,5 Stunden bei 40-44° wird die Reaktionsmischung auf Raumtemperatur gekühlt und mit Wasser verdünnt, wobei man die Temperatur unterhalb 35° hält. Die ausgefallene 3-(Benzthiazol-2-ylthio)-propan-1,2-dicarbonsäure wird abfiltriert, mit kaltem Wasser gewaschen und bei 60° im Vakuum getrocknet. Ausbeute 27,5 g (92 % d.Th.), Smp. 160-166°.

| | | | | | |
|---|---|---|---|---|---|
| Analyse ($C_{12}H_{11}NO_4S_2$) | ber. | 48,48 % C | 3,73 % H | 4,71 % N | 21,57 % S |
| | gef. | 48,2 % C | 3,7 % H | 4,6 % N | 21,3 % S |

Führt man die Addition von Itaconsäure an 2-Mercaptobenzthiazol analog Beispiel 3 in Trifluoressigsäure aus, so erhält man die 3-(Benzthiazol-2-ylthio)-propan-1,2-dicarbonsäure in 96 % Ausbeute.

Beispiel 5: In 100 ml Diethylether werden 13,3 g $AlCl_3$ bei 0 bis 5° gelöst. Dann werden 3,4 g 2-Mercaptobenzthiazol und 2,6 g Itaconsäure zugegeben. Nach 24 Stunden Rühren bei 20 - 25° wird die Etherlösung abgegossen und der Rückstand mit 100 ml Wasser und 100 ml Ethylacetat verrührt. Die organische Phase wird abgetrennt, getrocknet und eingedampft. Man erhält als Rückstand 3 g 3-(Benzthiazol-2-ylthio)-propan-1,2-dicarbonsäure.

Beispiel 6: In 50 ml 70 %iger $H_2SO_4$ werden 16,8 g 2-Mercaptobenzthiazol suspendiert und 13,7 g Glutaconsäure werden innerhalb 30 Minuten bei 45-50° unter Rühren zugeben. Das Reaktionsgemisch wird weitere 1,5 Stunden bei 45-50° gerührt und das Produkt wie in Beispiel 5 beschrieben isoliert. Man erhält 26 g (88 % d.Th.) 3-(Benzthiazol-2-ylthio)-glutarsäure, die bei 153-154° schmilzt.

| | | | | | |
|---|---|---|---|---|---|
| Analyse ($C_{12}H_4NO_4S_2$) | ber. | 48,48 % C, | 3,71 % H | 4,71 % N | 21,57 % S |
| | gef. | 48,5 % C, | 3,8 % H | 4,6 % N | 21,2 % S. |

Beispiel 7: 16,8 g fein pulverisiertes 2-Mercaptobenzthiazol werden in 50 ml 70 %iger wässriger Schwefelsäure suspendiert. Innerhalb von 30 Minuten werden unter gutem Rühren bei 45 bis 50° 9,0 g Methacrylsäure zugetropft. Das Gemisch wird während einer Stunde auf 50° gehalten und sodann bei 20-35° durch Zutropfen von 250 ml Wasser verdünnt. Das ausgeschiedene Produkt wird in Wasser unter Zusatz von Natronlauge gelöst und unlösliche Anteile durch Filtration entfernt. Aus dem Filtrat wird das Produkt durch Zugabe von Salzsäure ausgefällt und isoliert. Man erhält nach Trocknung im Vakuum bei 60° 22,3 g (88 % d.Th.) 3-(Benzthiazol-2-ylthio)-propan-2-carbonsäure, die nach Umkristallisation aus Cyclohexan/Hexan 1:1 bei 97 - 99° schmilzt.

| | | | | | |
|---|---|---|---|---|---|
| Analyse ($C_{11}H_{11}NO_2S_2$) | ber. | 52,15 % C | 4,38 % H | 5,53 % N | 25,31 % S |
| | gef. | 52,3 % C | 4,4 % H | 5,7 % N | 25,7 % S. |

In analoger Weise erhält man bei Verwendung von Crotonsäure anstelle von Methacrylsäure die 2-(Benzothiazol-2-ylthio)-propan-1-carbonsäure, die nach Umkristallisation aus Cyclohexan/Hexan 1:1 bei 61-63° schmilzt.

Beispiel 8: 47,4 g 2-Mercaptobenzimidazol und 40,7 g Itaconsäure werden in fein pulverisierter Form vermischt innerhalb einer Stunde eingetragen in 150 ml 70 %ige wässerige Schwefelsäure. Die Temperatur wird unter Rühren auf 40 bis 43° gehalten. Man lässt sodann während 1 1/2 Stunden bei obiger Temperatur ausreagieren und verdünnt die Reaktionsmischung mit Wasser und Eis auf ca. 2 Liter. Durch Zugabe von 30 %iger wässriger Natronlauge bis pH 4 wird das Produkt ausgefällt und abfiltriert. Nach Waschen mit Wasser und Trocknung im Vakuum bei ca. 30° erhält man 92,5 g 3-(Benzimidazol-2-ylthio)-propan-1,2-dicarbonsäure in Form eines Dihydrates vom Smp. 113° (Zers.), entsprechend 97 % d.Th.

Durch Umkristallisation aus Aceton wird das wasserfreie Produkt vom Smp. 165-168° erhalten.

Analyse ($C_{12}H_{12}N_2O_4S$) ber. 51,42 % C 4,32 % H 10,00 % N 11,44 % S
gef. 51,6 % C 4,4 % H 9,8 % N 11,2 % S.

In analoger Weise erhält man bei Verwendung von Maleinsäureanhydrid anstelle von Itaconsäure die Benzimidazol-2-ylthiobernsteinsäure, die bei 212° (unter Zersetzung) schmilzt.

Beispiel 9: 15,0 g 2-Mercaptobenzimidazol werden in 50 ml 70 %iger wässeriger Schwefelsäure gelöst. Innerhalb von ca. einer Stunde werden bei 20 bis 25° 9,0 g Acrylsäure zugetropft. Das Gemisch wird während 2 Stunden auf 20 bis 25° gehalten und sodann mit Eiswasser auf ca. 250 ml verdünnt. Durch Zugabe von Natronlauge wird der pH-Wert auf ca. 5 eingestellt, das Produkt abfiltriert, mit Wasser gewaschen und getrocknet. Man erhält 22,0 g (99 % d.Th.) 2-(Benzthiazol-2-ylthio)-propionsäure, die nach Umkristallisation aus Eissessig bei 188° (Zers.) schmilzt.

Analyse ($C_{10}H_{10}N_2O_2S$) ber. 54,04 % C 4,54 % H 12,6 % N 14,43 % S
gef. 54,3 % C 4,5 % H 12,5 % N 14,1 % S.

Verwendet man anstelle der Acrylsäure 10,8 g Methacrylsäure und führt die Reaktion bei 50° durch, so erhält man die 1-(Benzimidazol-2-ylthio)-propan-2-carbonsäure, Smp. 175-176°.

Verwendet man in analoger Weise 10,8 g Crotonsäure und führt die Reaktion bei 55° durch, so erhält man die 2-(Benzimidazol-2-ylthio)-propan-1-carbonsäure, die nach Umkristallisation aus Ethanol/Wasser 1:3 bei 153-155° schmilzt.

Beispiel 10: 4,45 g 2-Mercaptobenzthiazol und 5 g But-3-en-1,2,3-tricarbonsäure werden im Mörser gemeinsam verrieben. Das gepulverte Gemisch wird portionsweise unter Rühren in 60 ml 70 %ige $H_2SO_4$ bei 48-50° innerhalb einer Stunde eingetragen. Nach weiteren 3 Stunden bei 48-50°, kühlt man das Reaktionsgemisch auf Raumtemperatur und verdünnt unter Rühren mit 250 ml Wasser bei 25-30°. Die wässrige Lösung wird von etwas klebrigem Bodensatz abgegossen und mit weiteren 200 ml Wasser verdünnt. Nach 1 Stunde Rühren wird der Niederschlag abfiltriert und getrocknet. Die erhaltene 4-(Benzthiazol-2-ylthio)-butan-1,2,3-tricarbonsäure schmilzt nach Umkristallisation aus Methanol/Wasser bei 188-190°.

Analyse ($C_{14}H_{13}NO_6S_2$) ber. 47,32 % C 3,69 % H 3,94 % N
gef. 47,40 % C 3,86 % H 3,89 % N.

Beispiel 11: 8,6 g 6-Chlor-2-mercapto-4-methylbenzthiazol werden wie in Beispiel 1 beschrieben mit 4,4 g Maleinsäureanhydrid in 70 %iger $H_2SO_4$ bei 47-50° umgesetzt. Das Rohprodukt wird durch Umfällen aus $NaHCO_3$-Lösung gereinigt. Man erhält 6-Chlor-4-methylbenzthiazol-2-ylthiobernsteinsäure, die bei 168 - 171° unter Zersetzung schmilzt.

Analyse ($C_{12}H_{10}ClNO_4S_2$) ber. 43,44 % C 3,04 % H 4,22 % N
gef. 53,48 % C 3,20 % H 4,17 % N.

In analoger Weise erhält man aus 20 g 5-Carboxy-2-mercaptobenzthiazol und 10,26 g Maleinsäureanhydrid die 5-Carboxybenzthiazol-2-ylthiobernsteinsäure, die bei 210-215° unter Zersetzung schmilzt.

Analyse ($C_{12}H_9NO_6S_2$) ber. 44,04 % C 2,78 % H 4,28 % N
gef. 43,9 % C 2,78 % H 4,39 % N.

0126030



Patentansprüche

1. Verfahren zur Herstellung von Verbindungen der Formel I

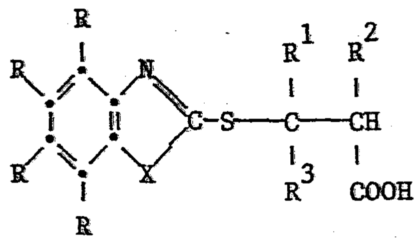

I ,

worin X Schwefel oder NH bedeutet, jedes R unabhängig von den anderen Wasserstoff, Alkyl, Halogenalkyl, Alkoxy, Alkylthio, Alkylsulfonyl, Phenyl, Alkylphenyl, Phenylalkyl, Cycloalkyl, Halogen, $-NO_2$, -CN, -COOH, -COOAlkyl, -OH oder eine primäre, sekundäre oder tertiäre Amino- oder Carbamoylgruppe bedeutet, und
$R^1$, $R^2$, $R^3$ und $R^4$ unabhängig voneinander Wasserstoff, Alkyl, Halogenalkyl, Hydroxyalkyl, Alkoxyalkyl, Carboxyalkyl, Carboxyl, unsubstituiertes oder substituiertes Aryl oder Aralkyl bedeuten oder
$R^1$ und $R^2$ zusammen eine direkte Bindung oder unverzweigtes oder verzweigtes Alkylen bedeuten, das durch 1 oder 2 Carboxylgruppen substituiert sein kann, durch Reaktion eines Mercaptans der Formel II

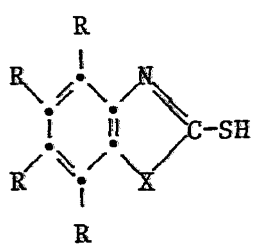

II

mit einer ungesättigten Carbonsäure der Formel III

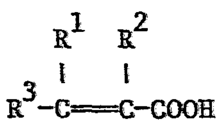

III

oder deren Anhydrid in stark saurem Medium.

2. Verfahren gemäss Anspruch 1, worin das Reaktionsmedium die wässrige oder organisch-wässrige Lösung einer starken Protonsäure ist.

3. Verfahren gemäss Anspruch 2, worin das Reaktionsmedium 60 - 90 %ige Schwefelsäure oder 25 - 38 %ige Salzsäure ist.

4. Verfahren gemäss Anspruch 1, dadurch gekennzeichnet, dass die Reaktion bei einer Temperatur von 0°C bis 100°C ausgeführt wird.

5. Verfahren gemäss Anspruch 1, dadurch gekennzeichnet, dass man ein Mercaptan der Formel II verwendet, worin mindestens zwei der Substituenten R Wasserstoff sind.

6. Verfahren gemäss Anspruch 5, worin ein R Wasserstoff, $C_1$-$C_4$-Alkyl, $C_1$-$C_4$-Alkoxy, Halogen, -COOH oder eine Aminogruppe ist und die anderen drei R Wasserstoff sind.

7. Verfahren gemäss Anspruch 1, dadurch gekennzeichnet, dass man eine Verbindung der Formel II verwendet, worin X Schwefel ist.

8. Verfahren gemäss Anspruch 1, dadurch gekennzeichnet, dass man eine Carbonsäure der Formel III verwendet, worin $R^1$, $R^2$ und $R^3$ unabhängig voneinander Wasserstoff, $C_1$-$C_8$-Alkyl, $C_2$-$C_8$-Carboxyalkyl, Carboxyl oder Phenyl bedeuten oder $R^1$ und $R^2$ zusammen Tetramethylen bedeuten.

9. Verfahren gemäss Anspruch 1, dadurch gekennzeichnet, dass man eine Carbonsäure der Formel III verwendet, worin mindestens zwei der Substituenten $R^1$, $R^2$ und $R^3$ Wasserstoff sind.

10. Verfahren gemäss Anspruch 1, dadurch gekennzeichnet, dass man

eine Carbonsäure der Formel III verwendet, worin $R^3$ Carboxyl ist oder $R^2$ Carboxymethyl ist, oder das cyclische Anhydrid einer solchen Verbindung.

11. Verfahren gemäss Anspruch 10, dadurch gekennzeichnet, dass man eine Verbindung der Formel II in stark saurem Medium mit Maleinsäure oder Maleinsäureanhydrid umsetzt.

FO 7.3/SA/rz*